# FASCICULE DE BREVET EUROPEEN

(11) **EP 2 104 510 B1**
(45) Date de publication et mention de la délivrance du brevet: **21.09.2011**
(21) Numéro de dépôt: 07872380.6
(22) Date de dépôt: 17.12.2007
(51) Int. Cl.: A61K 38/48, A61P 29/00

(54) **UTILISATION D'AU MOINS UNE NEUROTOXINE BOTULIQUE POUR TRAITER LA DOULEUR INDUITE PAR LES TRAITEMENTS THERAPEUTIQUES DU VIRUS DU SIDA**
VERWENDUNG VON MINDESTENS EINEM BOTULINUM-NEUROTOXIN ZUR BEHANDLUNG VON SCHMERZEN INFOLGE THERAPEUTISCHER BEHANDLUNGEN FÜR DAS AIDS-VIRUS
USE OF AT LEAST ONE BOTULISM NEUROTOXIN FOR TREATING THE PAIN INDUCED BY THERAPEUTIC TREATMENTS FOR THE AIDS VIRUS

(30) Priorité: 22.12.2006 FR 0611244
(43) Date de publication de la demande: 30.09.2009
(73) Titulaire: IPSEN PHARMA S.A.S., 92100 Boulogne-Billancourt (FR)
(72) Inventeur: Favre, Christine, F-91530 Saint Maurice Montcouronne (FR); Auguet, Michel, F-91120 Palaiseau (FR); Chabrier De Lassauniere, Pierre-Etienne, F-75016 Paris (FR)
(74) Mandataire: Retzler, Charlotte
(86) Numéro de dépôt international: PCT/FR2007/002091
(87) Numéro de publication internationale: WO 2008/090287

(56) Documents cités:
- KLEIN ARNOLD WILLIAM: "The therapeutic potential of botulinum toxin." DERMATOLOGIC SURGERY : OFFICIAL PUBLICATION FOR AMERICAN SOCIETY FOR DERMATOLOGIC SURGERY [ET AL.] MAR 2004, vol. 30, no. 3, mars 2004 (2004-03), pages 452-455, XP002450524 ISSN: 1076-0512
- NOGUERA ET AL: "Botulinum toxin in the treatment of spasticity in HIV-infected children affected with progressive encephalopathy" AIDS, LONDON, GB, vol. 18, no. 2, 2004, pages 352-353, XP003001918 ISSN: 0269-9370
- LIU HSU-TANG ET AL: "Botulinum toxin A relieved neuropathic pain in a case of post-herpetic neuralgia." PAIN MEDICINE (MALDEN, MASS.) 2006 JAN-FEB, vol. 7, no. 1, janvier 2006 (2006-01), pages 89-91, XP002450525 ISSN: 1526-2375
- BACH-ROJECKY L ET AL: "Botulinum toxin type A in experimental neuropathic pain" JOURNAL OF NEURAL TRANSMISSION ; BASIC NEUROSCIENCES, GENETICS AND IMMUNOLOGY, PARKINSON'S DISEASE AND ALLIED CONDITIONS, ALZHEIMER'S DISEASE AND ADOLESCENT PSYCHIATRY RELATED DISORDERS, BIOLOGICAL PSYCHIATRY, BIOLOGICAL CHILD AND ADOLESCENT PSYCHIAT, vol. 112, no. 2, 1 février 2005 (2005-02-01), pages 215-219, XP019378044 ISSN: 1435-1463
- FRICH L M ET AL: "Pain and pain treatment in AIDS patients: a longitudinal study." JOURNAL OF PAIN AND SYMPTOM MANAGEMENT MAY 2000, vol. 19, no. 5, mai 2000 (2000-05), pages 339-347, XP002450526 ISSN: 0885-3924
- LUCIANO CARLOS A ET AL: "Recent developments in the HIV neuropathies.", CURRENT OPINION IN NEUROLOGY JUN 2003 LNKD- PUBMED:12858079, vol. 16, no. 3, June 2003 (2003-06), pages 403-409, ISSN: 1350-7540
- JOSEPH ELIZABETH K ET AL: "Novel mechanism of enhanced nociception in a model of AIDS therapy-induced painful peripheral neuropathy in the rat.", PAIN JAN 2004 LNKD- PUBMED:14715401, vol. 107, no. 1-2, January 2004 (2004-01), pages 147-158, ISSN: 0304-3959
- KESWANI SANJAY C ET AL: "HIV-associated sensory neuropathies.", AIDS (LONDON, ENGLAND) 8 NOV 2002 LNKD- PUBMED:12409731, vol. 16, no. 16, 8 November 2002 (2002-11-08), pages 2105-2117, ISSN: 0269-9370
- LUCIANO CARLOS A ET AL: "Recent developments in the HIV neuropathies.", CURRENT OPINION IN NEUROLOGY JUN 2003 LNKD- PUBMED:12858079, vol. 16, no. 3, June 2003 (2003-06), pages 403-409, ISSN: 1350-7540
- JOSEPH ELIZABETH K ET AL: "Novel mechanism of enhanced nociception in a model of AIDS therapy-induced painful peripheral neuropathy in the rat.", PAIN JAN 2004 LNKD- PUBMED:14715401, vol. 107, no. 1-2, January 2004 (2004-01), pages 147-158, ISSN: 0304-3959
- KESWANI SANJAY C ET AL: "HIV-associated sensory neuropathies.", AIDS (LONDON, ENGLAND) 8 NOV 2002 LNKD- PUBMED:12409731, vol. 16, no. 16, 8 November 2002 (2002-11-08), pages 2105-2117, ISSN: 0269-9370

## Description

La présente invention a pour objet l'utilisation d'au moins une neurotoxine botulique pour l'obtention d'un médicament destiné à traiter ou prévenir la ou les douleurs provoquées par les médicaments anti-VIH caractérisée en ce que la ou les douleurs à traiter ou prévenir sont des douleurs provoquées par les médicaments anti-VIH administrés aux patients atteints du SIDA.

Le SIDA reste encore aujourd'hui une pathologie difficile à soulager et à guérir. De plus les traitements thérapeutiques du SIDA actuellement disponibles ont pour effet secondaire de provoquer des douleurs, parmi ces douleurs on peut citer entre autres les douleurs nociceptives, neuropathiques ou idiopathiques.

Plusieurs études ont été conduites dans l'utilisation de la toxine botulique pour atténuer ou prévenir différentes pathologies liées au VIH. Ces différentes utilisations de la toxines sont décrites dans la littérature scientifique et médicale.

L'utilisation de la toxine botulique comme analgésique pour les douleurs engendrées par le VIH lui-même est connue de la publication scientifique Klein A.W. " The therapeutic potential of botulinum toxin ", Dermatologic Surgery, vol 30, no. 3, mars 2004, pages 452-455.

La toxine botulique utilisée dans le cadre de traitement d'enfants atteints du VIH et souffrant conjointement de paralysie spasmodique ainsi que de paralysie cérébrale liée au VIH est décrite dans la publication scientifique de Noguera et AL "Botulinium toxin in the treatment of spasticity in HIV-infected children affected with progressive encephalopaty", AIDS, vol. 18, no. 2, 2004, pages 352-353.

Une étude sur l'utilisation de la toxine botulique dans le cas de névralgies post-herpétiques est relatée dans la publication scientifique de Liu HSU-TANG et Al "Botulinum toxin A a relieved neuropathic pain in a case of post herpetic neuralgia" Pain Medicine, 2006, vol.7, no. 1, janvier 2006, pages 89-91.

La publication de Bach-Rojeckey L et Al "Botulinium toxin type A in experimental neuropathic pain" Journal of neural transmission, vol. 112, no.2, 1er février 2005, pp. 215-219., décrit une réduction de l'hyperalgésie mécanique et thermique grâce à l'utilisation de la toxine botulique dans le cadre particulier d'une expérience effectuée sur des rats, qui ont préalablement subi une section partielle transversale du nerf sciatique.

Parmi les douleurs les plus fréquentes chez les patients atteints du SIDA et provoquées par les médicaments anti-VIH on peut citer les douleurs abdominales, les céphalées, les neuropathies périphériques, les myalgies ou les arthralgies, cette liste n'étant pas exhaustive.

Ces douleurs touchent de nombreux patients infectés par le VIH et traités de manière chronique, par exemple pendant plus d'un an, par un traitement chimique pour lutter contre le SIDA. Ce type de douleur est à distinguer de la douleur provoquée par le virus lui-même. En effet ces douleurs sont induites par le ou les médicaments anti-VIH administrés aux patients aux fins de les soigner.

Les douleurs attribuables aux médicaments anti-VIH ont des caractéristiques sémiologiques particulières. Par exemple, la neuropathie périphérique se caractérise par une douleur continue, diffuse, sans rythme mécanique ou inflammatoire, du type brûlure. Sur ce fond douloureux continu peuvent survenir d'autres symptômes : des accès spontanés du type élancements, picotements plus particulièrement des picotements au niveau des extrémités des membres, ou encore des décharges électriques. La topographie de ces symptômes correspond à une distribution compatible avec une systématisation périphérique ou centrale. En d'autres termes la topographie de ces douleurs neuropathiques attribuables aux médicaments anti-VIH est indépendante de la topographie de l'infection par le virus.

Parmi les traitements connus de cette douleur, on peut aussi citer par exemple l'administration d'anticonvulsivants, d'anti-dépresseurs ou de composés opiacés tels que la morphine.

Cependant, l'usage des composés actuellement disponibles qui permettent de réduire la douleur provoquée par un traitement avec des médicaments anti-VIH n'est pas satisfaisant car il nécessite l'emploi de doses élevées de composés, ou bien une ré-administration fréquente du composé avec un possible développement d'une résistance au composé ou accoutumance. De plus ces traitements anti-douleurs peuvent provoquer des effets secondaires, qui s'ajoutent à ceux déjà provoqués par le SIDA ou par le traitement de celui-ci.

Or l'impact des souffrances engendrées a souvent un effet dévastateur sur la qualité de la vie des personnes atteintes.

Il est donc devenu nécessaire de trouver un autre moyen pour traiter ou prévenir les douleurs provoquées par les médicaments anti-VIH.

Aussi le problème que se propose de résoudre l'invention est de trouver un nouveau traitement de la douleur provoquée par les traitements anti-VIH, notamment les traitements antirétroviraux.

Dans ce but la présente invention propose l'utilisation d'au moins une neurotoxine botulique pour l'obtention d'un médicament destiné à traiter ou prévenir la ou les douleurs provoquées par les médicaments anti-VIH caractérisée en ce que la ou les douleurs à traiter ou prévenir sont des douleurs provoquées par les médicaments anti-VIH administrés aux patients atteints du SIDA.

L'invention offre des avantages déterminants, en particulier celui d'éviter ou de prévenir la douleur suite à un traitement par un médicament anti-VIH et ainsi permettre l'augmentation des doses de traitement anti-VIH sans augmenter la douleur.

Avantageusement, l'invention permet de prévenir ou soulager la douleur sans interférer avec l'efficacité du traitement anti-VIH.

Enfin l'invention a pour avantage de pouvoir être mise en oeuvre dans toutes industries, notamment l'industrie pharmaceutique et cosmétique.

D'autres avantages et caractéristiques de l'invention apparaîtront clairement à la lecture de la description et des exemples donnés à titre purement illustratifs et non limitatifs qui vont suivre.

Par " douleur " au sens de la présente invention, il faut entendre " toute expérience désagréable émotionnelle et sensorielle associée à un dommage tissulaire présent ou potentiel, ou décrite par le patient en de tels termes ".

Par l'expression neurotoxine botulique, on entend une toxine botulique qui est soit une protéine libre (i.e. libre de toute protéine la complexant), soit un complexe protéique, ledit complexe protéique pouvant comprendre par exemple de l'hémagglutinine (protéine HA) associée à de la toxine botulique, ou soit un fragment protéique.

Par l'expression toxine botulique, on entend une molécule possédant l'activité biologique de la toxine botulique, qui peut être par exemple soit une protéine, soit un polypeptide, soit un peptide, soit une protéine de fusion, soit une protéine tronquée, soit une protéine chimérique, soit une protéine mutée ou une protéine recombinante.

Par l'expression activité biologique de la toxine botulique, on entend au sens de la présente invention soit une paralysie musculaire soit une inhibition de l'exocytose, en particulier de l'exocytose de l'acétylcholine ou d'un autre neurotransmetteur.

Par protéine, polypeptide ou peptide on entend au sens de la présente invention, un polymère d'acides aminés, naturels ou non, lévogyres ou non, dextrogyres ou non.

Par protéine chimérique, on entend au sens de la présente invention une protéine obtenue après association de différents types de molécules, par exemple après association de lipides, de glycolipides, de peptides, de polypeptides, de protéines, de glycoprotéines, de carbohydrates, de polysaccharides, d'acides nucléiques, de polyéthylène glycol, etc.

La toxine botulique, en particulier la toxine botulique de type A1 (Dysport^{®} commercialisé par Ipsen ou Botox^{®} commercialisé par Allergan), est utilisée depuis les années 80 chez l'homme pour le traitement de maladies / désordres divers et variés. Parmi les maladies / désordres pouvant être traités par la toxine botulique, on peut citer entre autres des désordres musculaires (par exemple le blépharospasme, la spasticité de l'adulte ou de l'enfant ou encore le torticolis, le strabisme), l'hyperhydrose (ou transpiration excessive), l'hypersalivation ou même les rides de la glabelle.

La neurotoxine botulique, pure ou quasiment pure, peut être obtenue à partir d'un complexe protéique comprenant de la toxine botulique par exemple selon la méthode décrite dans Current topics in Microbiology and Immunology (1995), 195, p. 151-154. Une neurotoxine botulique, pure ou quasiment pure, peut être obtenue par exemple, par purification d'un milieu de fermentation ou bouillon de culture contenant une souche de *Clostridium Botulinum,* et enrichi par exemple en viande ou en nourriture protéinée.

La présente invention a tout d'abord pour objet l'utilisation d'au moins une neurotoxine botulique pour l'obtention d'un médicament destiné à traiter ou prévenir la ou les douleurs provoquées par les médicaments anti-VIH administrés aux patients atteints du SIDA.

De préférence, la présente invention n'a pas pour objet de traiter le VIH c'est-à-dire le virus en lui-même, ni de traiter ou prévenir la ou les douleurs provoquées par le virus en lui-même ou par la maladie elle-même.

L'invention a pour objet l'utilisation d'au moins une neurotoxine botulique pour l'obtention d'un médicament destiné à traiter ou prévenir la ou les douleurs provoquées par les médicaments anti-VIH et non pas la ou les douleurs provoquées par le VIH ou la maladie due au VIH.

Plus préférentiellement, la ou les douleurs à traiter ou prévenir selon l'invention sont provoquées par des médicaments anti-VIH choisis parmi les inhibiteurs de la transcriptase inverse, les antiprotéases, les inhibiteurs de fusion, les cytotoxiques ou les interleukines.

De préférence, le ou les médicaments anti-VIH sont choisis parmi les composés suivants ou leurs mélanges :
- zidovudine (AZT), didanosine, stavudine, zalcitabine, lamivudine, néviparine, abacadir, emtricitabine (pour les inhibiteurs de la transférase inverse) ;
- saquinavir, ritonavir, indinavir, nelfinavir, amprenavir, tenofovir, atazanavir, le mélange de lopinavir, ritonavir et fosamprenavir (pour les antiprotéases) ;
- enfuvirtide (pour les inhibiteurs de fusion)
- IL2 (Macrolin) (pour les interleukines)
- ou le mélange de 1 ou 2 inhibiteurs de transcriptase inverse avec 1 ou 2 antiprotéases (trithérapie).

D'autres médicaments à but anti-VIH non spécifiques sont également utilisés pour traiter les maladies opportunistes de l'infection. Par exemple, on peut citer la thalidomide, le dapsone et certains antibiotiques comme le metronidazole ou l'isoniazide.

Aussi l'invention a également pour objet l'utilisation d'au moins une neurotoxine botulique pour traiter ou prévenir la ou les douleurs provoquées par des médicaments traitant les maladies opportunistes de l'infection par le VIH, choisis parmi les composés suivants ou leurs mélanges : la thalidomide, le dapsone et certains antibiotiques comme le metronidazole ou l'isoniazide.

De manière préférentielle, la neurotoxine botulique permet d'obtenir un effet systémique.

Par " effet systémique ", on entend au sens de la présente invention une administration localisée permettant d'obtenir un effet généralisé.

Il est également possible d'obtenir un effet localisé lorsque la douleur est de préférence localisée.

Selon une utilisation préférée de l'invention la neurotoxine botulique est administrée par voie sous-cutanée, intra-musculaire ou intra-thécale.

De préférence la neurotoxine botulique utilisée selon l'invention est choisie parmi les neurotoxines botuliques de type A, A1, A2, B, C, C1, D, E, F ou G.

La neurotoxine botulique de type A1 correspond en fait à la toxine botulique classique qui est communément appelée toxine botulique de type A, sans distinction du sous-type. La neurotoxine botulique de type A1 est commercialisée sous le nom de DYSPORT^{®} ou BOTOX^{®}.

Selon l'invention, la neurotoxine botulique de type A1 peut correspondre soit à un complexe de toxine botulique A1 et d'hémagglutinine, soit à la toxine botulique de type A1 libre de toutes protéines complexantes.

La toxine botulique de type A2 a d'abord été isolée à partir de cas d'enfants atteints de botulisme vers 1990 (Sakaguchi et al., Int. J. Food Microbiol. (1990), 11, 231-242). Cette toxine est immunologiquement et biochimiquement différente de la toxine botulique de type A1.

La toxine botulique de type A2 peut être isolée à partir des souches suivantes : Kyoto-F, Chiba-H, Y-8036, 7103-H, 7105-H, KZ1828, NCTC2012 ou NCTC9837 (Cordoba et al., System. Appl. Microbiol. (1995), 18, 13-22; Franciosa et al., abstract presented at 40th Interagency Botulism Research Coordinating Committee (IBRCC) Meeting, November 2003).

De préférence la neurotoxine botulique utilisée selon l'invention est la toxine botulique de type A1.

Selon une variante de l'invention, la neurotoxine botulique utilisée selon l'invention est la toxine botulique de type A2 isolée à partir de la souche Clostridium botulinum référencée et accessible sous le numéro NCTC9837, auprès de la National Collection of Type Cultures - Central Public Health Laboratory - London - UK. La souche NCTC9837 est parfois appelée souche Mauritius 1955.

La toxine botulique de type A2 diffère de la toxine A1 par, entre autres, sa séquence en acides aminés, son poids moléculaire, ses caractéristiques immunologiques et génétiques (Kubota et al., Biochem. Biophys. Res. Commun. (1996), 224 (3), 843-848).

Selon un mode préférentiel, la neurotoxine botulique utilisée selon l'invention est une neurotoxine botulique modifiée ayant au moins un acide aminé supprimé, modifié ou remplacé.

De préférence la neurotoxine botulique utilisée selon l'invention est associée à au moins un polysaccharide ou un mélange de plusieurs polysaccharides.

Par polysaccharide, on entend au sens de la présente invention, un polymère comprenant au moins 2 monomères, les monomères étant des saccharides. Cette définition inclut les disaccharides.

Dans le cadre de l'invention, les polysaccharides peuvent être ioniques et/ou non ioniques.

De préférence, la composition comprend au moins un polysaccharide comprenant majoritairement des unités de glucose. Le terme majoritairement signifiant que le glucose est majoritaire en nombre d'unités de monomère.

Comme exemple de polysaccharides convenant selon l'utilisation de l'invention, on peut citer l'amidon, les dérivés d'amidon, l'hydroxyéthyle amidon et en particulier le 2-hydroxy-éthyl amidon.

Les polysaccharides convenant selon la présente invention peuvent être substitués, en particulier peuvent être substitués par des radicaux alkyles, alcoxy, ou encore par des radicaux alkyles eux-mêmes substitués par des fonctions alcools.

Selon une variante de l'invention, la quantité de polysaccharide convenant selon la présente invention est d'au moins 1 µg de polysaccharide pour 1 unité de toxine botulique. Selon le choix du polysaccharide, il est possible d'utiliser au moins 0,5 µg de polysaccharide pour 1 unité de toxine botulique.

De préférence la neurotoxine botulique utilisée selon l'invention est associée à au moins un tensio-actif ou un mélange de plusieurs tensio-actifs.

Par agent tensio-actif, on entend au sens de l'invention un agent émulsifiant ou un agent solubilisant.

Dans le cadre de l'invention, les tensio-actifs mis en oeuvre peuvent être choisis parmi les tensio-actifs cationiques, anioniques ou non-ioniques.

De préférence la neurotoxine botulique utilisée selon l'invention est associée à au moins un tensio-actif ou un mélange de plusieurs tensio-actifs, choisi parmi les tensio-actifs cationiques, anioniques ou non-ioniques.

De préférence la neurotoxine botulique utilisée selon l'invention est associée à au moins un tensio-actif choisi parmi les tensio-actifs non-ioniques du groupe des polysorbates.

Parmi le groupe des polysorbates, on peut citer le polysorbate 20, le polysorbate 21, le polysorbate 40, le polysorbate 60, le polysorbate 61, le polysorbate 65, le polysorbate 80, le polysorbate 81, le polysorbate 85, le polysorbate 120, le polysorbate 80 acetate.

Le tensio-actif préféré selon une variante de l'invention est le polysorbate 80.

La neurotoxine botulique utilisée selon l'invention peut être administrée de préférence par injection comme par exemple par injection intramusculaire, intra-thécale ou sous-cutanée.

Dans le cas des injections selon l'invention, la neurotoxine botulique pourra être associée à un agent facilitant l'injection encore appelé véhicule d'injection ou vecteur d'injection.

La dose d'utilisation selon la présente invention de la neurotoxine botulique, à prévoir pour le traitement ou la prévention des maladies ou troubles mentionnés ci-dessus, varie suivant le mode d'administration, l'âge et le poids corporel du sujet à traiter ainsi que l'état de santé de ce dernier, et il en sera décidé en définitive par le médecin traitant. Une telle quantité déterminée par le médecin traitant est appelée ici "quantité thérapeutiquement efficace".

De préférence, la neurotoxine botulique utilisée selon l'invention est administrée à une dose comprise entre 0,01 U et 1500 U, préférentiellement à une dose comprise entre 0,01 U et 1000 U, plus préférentiellement de 0,1 U à 500 U, plus particulièrement à une dose comprise entre 0,1 U et 100 U, encore plus particulièrement à une dose comprise entre 1 et 20 U, et ceci quel que soit le type de toxine botulique ou quelle que soit sa provenance.

L'unité de toxine (U) est définie ci-après dans la partie exemple.

La présente invention a pour objet l'utilisation de la neurotoxine botulique, décrite ci-dessus, pour l'obtention d'un médicament destiné à traiter ou prévenir la ou les douleurs provoquées par les médicaments anti-VIH, c'est-à-dire les douleurs liées à un traitement contre le SIDA.

L'abréviation " SIDA" signifie Syndrome de l'Immunodéficience Acquise. Par l'expression " SIDA ", on entend au sens de la présente invention tout type d'infection par le virus de l'immunodéficience humaine, également nommé " VIH " en français ou " HIV " en anglais.

Par l'expression " patient atteint du SIDA ", on entend au sens de l'invention aussi bien un patient séropositif, c'est-à-dire contaminé par le virus, qu'un patient développant les symptômes de cette maladie ou des maladies opportunistes de l'infection.

Selon une utilisation préférée de l'invention la neurotoxine botulique permet de traiter ou prévenir la ou les douleurs induites par les traitements antirétroviraux ou les traitements des maladies opportunistes de l'infection par le VIH.

L'exemple suivant illustre l'invention sans en limiter la portée.

### EXEMPLE

La quantification des neurotoxines botuliques utilisées selon l'invention a été réalisée par la mesure d'une dose létale DL₅₀. Par DL₅₀ on entend au sens de la présente invention la dose létale ou encore dose semi létale d'une substance donnée. Il s'agit de la dose (ou quantité) qui conduit à la mort de 50% des animaux testés d'un groupe. Une unité de toxine (U) correspond à la DL₅₀ chez la souris par voie intra péritonéale.

Dans l'exemple 1, la toxine botulique employée est la neurotoxine botulique de type A1 commercialisée sous la marque Dysport^{®}.

Exemple 1 : un patient mâle de 31 ans, HIV séropositif et traité contre le SIDA par une trithérapie avec des antirétroviraux, présentait des douleurs sévères dans le bras gauche, des fourmillements dans les membres inférieurs et supérieurs ainsi que des brûlures et une hypersensibilité au toucher. Cette hypersensibilité au toucher se traduisait au niveau des mains par le fait qu'il ne pouvait enfiler des gants car cela lui était insupportable. Ces symptômes douloureux, caractéristiques des trithérapies, avaient été provoqués par la trithérapie avec des antirétroviraux et correspondaient à des effets secondaires du traitement. Le patient a alors reçu 3 injections sous-cutanées de 10 U chacune de Dysport^{®} dans le bras gauche. Après 48 heures les douleurs avaient totalement disparues.

## Revendications

1. Utilisation d'au moins une neurotoxine botulique pour l'obtention d'un médicament destiné à traiter ou prévenir la ou les douleurs provoquées par les médicaments anti-VIH **caractérisée en ce que** la ou les douleurs à traiter ou prévenir sont des douleurs provoquées par les médicaments anti-VIH administrés aux patients atteints du SIDA.

2. Utilisation selon la revendication précédente **caractérisée en ce que** la ou les douleurs à traiter ou prévenir sont provoquées par des médicaments anti-VIH choisis parmi les inhibiteurs de la transcriptase inverse, les antiprotéases, les inhibiteurs de fusion, les cytotoxiques ou les interleukines.

3. Utilisation selon l'une des revendications précédentes **caractérisée en ce que** la ou les douleurs à traiter ou prévenir sont provoquées par des médicaments anti-VIH choisis parmi les composés suivants ou leurs mélanges : zidovudine (AZT), didanosine, stavudine, zalcitabine, lamivudine, néviparine, abacadir, emtricitabine, saquinavir, ritonavir, indinavir, nelfinavir, amprenavir, tenofovir, atazanavir, enfuvirtide, IL2 (Macrolin) ou le mélange de lopinavir, ritonavir et fosamprenavir.

4. Utilisation selon l'une des revendications précédentes **caractérisée en ce que** la ou les douleurs à traiter ou prévenir sont provoquées par des médicaments traitant les maladies opportunistes de l'infection par le VIH choisis parmi les composés suivants ou leurs mélanges : la thalidomide, le dapsone et certains antibiotiques comme le metronidazole ou l'isoniazide.

5. Utilisation selon l'une des revendications précédentes **caractérisée en ce que** la neurotoxine botulique permet d'obtenir un effet systémique.

6. Utilisation selon l'une des revendications précédentes **caractérisée en ce que** la neurotoxine botulique est administrée par voie sous-cutanée, intra-musculaire ou intra-thécale.

7. Utilisation selon l'une des revendications précédentes **caractérisée en ce que** la neurotoxine botulique est choisie parmi les neurotoxines botuliques de type A, A1, A2, B, C, C1, D, E, F ou G.

8. Utilisation selon l'une des revendications précédentes **caractérisée en ce que** la neurotoxine botulique est la toxine botulique de type A1.

9. Utilisation selon l'une des revendications précédentes **caractérisée en ce que** la neurotoxine botulique est associée à au moins un polysaccharide ou un mélange de plusieurs polysaccharides.

10. Utilisation selon la revendication 9 **caractérisée en ce que** le polysaccharide est le 2-hydroxy-ethyl amidon.

11. Utilisation selon l'une des revendications précédentes **caractérisée en ce que** la neurotoxine botulique est une neurotoxine botulique modifiée ayant au moins un acide aminé supprimé, modifié ou remplacé.

12. Utilisation selon l'une des revendications précédentes **caractérisée en ce que** la neurotoxine botulique est associée à au moins un tensio-actif ou un mélange de plusieurs tensio-actifs, choisis parmi les tensio-actifs cationiques, anioniques ou non-ioniques.

13. Utilisation selon l'une des revendications précédentes **caractérisée en ce que** la neurotoxine botulique est associée à au moins un tensio-actif choisi parmi les tensio-actifs non-ioniques du groupe des polysorbates.

14. Utilisation selon l'une des revendications précédentes **caractérisée en ce que** la neurotoxine botulique est administrée à une dose comprise entre 0,01 U et 1500 U.

## Claims

1. Use of at least one botulinum neurotoxin to obtain a medicament for treating or preventing pain induced by anti-HIV medicaments, **characterized in that** the pain to be treated or prevented is pain induced by anti-HIV medicaments administered to patients suffering from AIDS.

2. Use according to the preceding claim, **characterized in that** the pain to be treated or prevented is induced by anti-HIV medicaments chosen from reverse transcriptase inhibitors, antiproteases, fusion inhibitors, cytotoxics and interleukins.

3. Use according to any one of the preceding claims, **characterized in that** the pain to be treated or prevented is induced by anti-HIV medicaments chosen from the following compounds or mixtures thereof: zidovudin (AZT), didanosin, stavudin, zalcitabin, lamivudin, neviparin, abacadir, emtricitabin, saquinavir, ritonavir, indinavir, nelfinavir, amprenavir, tenofovir, atazanavir, enfuvirtide, IL2 (Macrolin) or the mixture of lopinavir, ritonavir and fosamprenavir.

4. Use according to any one of the preceding claims, **characterized in that** the pain to be treated or prevented is induced by a medicament treating opportunistic diseases of a HIV infection, which is chosen amongst the following compounds or mixtures thererof: thalidomide, dapsone and certain antibiotics such as metronidazole or isoniazid.

5. Use according to any one of the preceding claims, **characterized in that** the botulinum neurotoxin allows obtaining a systemic effect.

6. Use according to any one of the preceding claims, **characterized in that** the botulinum neurotoxin is administered by sub-cutaneous, intra-muscular or intra-thecal route.

7. Use according to any one of the preceding claims, **characterized in that** the botulinum neurotoxin is chosen from botulinum toxin type A, A1, A2, B, C, C1, D, E, F or G.

8. Use according to any one of the preceding claims, **characterized in that** the botulinum neurotoxin is botulinum toxin type A1.

9. Use according to any one of the preceding claims, **characterized in that** the botulinum neurotoxin is combined with at least one polysaccharide or a mixture of several polysaccharides.

10. Use according to claim 9, **characterized in that** the polysaccharide is 2-hydroxyethyl starch.

11. Use according to one of the preceding claims, **characterized in that** the botulinum neurotoxin is a modified botulinum neurotoxin having at least one deleted, modified or replaced amino acid.

12. Use according to any one of the preceding claims, **characterized in that** the botulinum neurotoxin is combined with at least one surfactant or a mixture of several surfactants, chosen from cationic, anionic or non-ionic surfactants.

13. Use according to any one of the preceding claims, **characterized in that** the botulinum neurotoxin is combined with at least one surfactant chosen from non-ionic surfactants of the polysorbate group.

14. Use according to any one of the preceding claims, **characterized in that** the botulinum neurotoxin is administered at a dose comprised between 0.01 U and 1500 U.

## Patentansprüche

1. Verwendung zumindest eines Botulinumneurotoxins zum Erhalt eines Medikaments zur Behandlung oder Verhütung von Schmerzen, die durch anti-HIV Medikamente ausgelöst werden, **dadurch gekennzeichnet, dass** die durch Anti-HIV-Medikamente ausgelösten Schmerzen, die behandelt oder verhütet werden sollen, einem Patienten verabreicht werden, der an AIDS erkrankt ist.

2. Verwendung nach dem vorangehenden Anspruch, **dadurch gekennzeichnet, dass** die Schmerzen, die behandelt oder verhütet werden sollen, durch Anti-HIV-Medikamente ausgelöst werden, die ausgewählt sind unter Reverse-Transkriptase-Inhibitoren, Antiproteasen, Fusionsinhibitoren, cytotoxischen Mitteln und Interleukinen.

3. Verwendung nach einem der vorangehenden Ansprüche, **dadurch gekennzeichnet, dass** die Schmerzen, die behandelt oder verhütet werden sollen, durch solche Anti-HIV-Medikamente ausgelöst werden, die unter den folgenden Verbindungen, oder Gemischen davon, ausgewählt sind: Zidovudin (AZT), Didanosin, Stavudin, Zalcitabin, Lamivudin, Neviparin, Abacadir, Emtricitabin, Saquinavir, Ritonavir, Indinavir, Nelfinavir, Amprenavir, Tenofovir, Atazanavir, Enfuvirtide, IL2 (Macrolin) oder einem Gemisch aus Lopinavir, Ritonavir und Fosamprenavir.

4. Verwendung nach einem der vorangehenden Ansprüche, **dadurch gekennzeichnet, dass** die Schmerzen, die behandelt oder verhütet werden sollen, durch ein Medikament ausgelöst werden, welches zur Behandlung von opponunistischen Krankheiten von HIV Infektionen verwendet wird, das ausgewählt ist unter den folgenden Verbindungen, oder Gemischen davon: Thalidomid, Dapson und bestimmte Antibiotika wie zum Beispiel Metronidazol oder Isoniazid.

5. Verwendung nach einem der vorangehenden Ansprüche, **dadurch gekennzeichnet, dass** das Botulinumneurotoxin es erlaubt, einen systemischen Effekt zu erzielen.

6. Verwendung nach einem der vorangehenden Ansprüche, **dadurch gekennzeichnet, dass** das Botulinumneurotoxin subkutan, intermuskulär oder intrathekal verabreicht wird.

7. Verwendung nach einem der vorangehenden Ansprüche, **dadurch gekennzeichnet, dass** das Botulinumneurotoxin ausgewählt ist unter Botulinumtoxin Typ A, A1, A2, B, C, C1, D, E, F oder G.

8. Verwendung nach einem der vorangehenden Ansprüche, **dadurch gekennzeichnet, dass** das Botulinumneurotoxin Botulinumtoxin Typ A1 ist.

9. Verwendung nach einem der dem vorangehenden Ansprüche, **dadurch gekennzeichnet, dass** das Botulinumneurotoxin mit zumindest einem Polysaccharid oder einem Gemisch mehrerer Polysaccharide kombiniert wird.

10. Verwendung nach Anspruch 9, **dadurch gekennzeichnet, dass** das Polysaccharid 2-Hydroxyethylstärke ist.

11. Verwendung nach einem der vorangehenden Ansprüche, **dadurch gekennzeichnet, dass** das Botulinumneurotoxin ein modifiziertes Botulinumneurotoxin ist, in welchem zumindest eine Aminosäure deletiert, modifiziert oder ersetzt ist.

12. Verwendung nach einem der vorangehenden Ansprüche, **dadurch gekennzeichnet, dass** das Botulinumneurotoxin mit zumindest einem Tensid oder einem Gemisch mehrerer Tenside kombiniert wird, die ausgewählt sind unter kationischen, anionischen oder nichtionischen Tensiden.

13. Verwendung nach einem der vorangehenden Ansprüche, **dadurch gekennzeichnet, dass** das Botulinumneurotoxin mit zumindest einem Tensid kombiniert wird, welches ausgewählt ist under den nichtionischen Tensiden der Polysorbatgruppe.

14. Verwendung nach einem der vorangehenden Ansprüche, **dadurch gekennzeichnet, dass** das Botulinumneurotoxin in einer Dosis verabreicht wird, die zwischen 0,01 U und 1500 U liegt.
